# EUROPEAN PATENT APPLICATION

(11) **EP 1 172 353 A1**
(43) Date of publication of application: **16.01.2002**
(21) Application number: 00870158.3
(22) Date of filing: 10.07.2000
(51) Int. Cl.: C07C 51/50, C07C 55/14, C07C 55/02, C11D 17/00, C11D 3/20

(54) **Retardation of discolouration of dicarboxylic acids**

(71) Applicant: The Procter & Gamble Company, Cincinnati, Ohio 45202 (US)
(72) Inventor: Van Dijck, Paul Irma Albertus, 2580 Putte (BE)
(74) Representative: Mather, Peter Geoffrey

(57) **Abstract**

According to the present invention there is provided a process for retarding the discolouration of dicarboxylic acids, said process comprising:
(a) heating said dicarboxylic acid to a temperature above its melting point;
(b) adding water to said dicarboxylic acid.

A further aspect of the present invention provides a composition for retarding the discolouration of dicarboxylic acids, said composition comprising 1g, preferably 2.5g, more preferably 5g, of water per 1,000g of dicarboxylic acid. A further aspect of the present invention provides for the use of water to retard the discolouration of dicarboxylic acids, particularly molten dicarboxylic acids.

## Description

### Technical Field

The present invention relates to the retardation of the discolouration of dicarboxylic acids and, in particular, the retardation of the discolouration of molten dicarboxylic acids.

### Background of the Invention

Dicarboxylic acids are used in a variety of ways and are especially valued in the polymer industry where they are used in the production of various polymers including polyamides and polyurethanes. More recently dicarboxylic acids have been found to be useful in the coating of laundry and dishwashing tablets (see, for example, EP-A-846,754).

Many of the uses of dicarboxylic acids require that they be molten during at least part of the process. For example, when hexamethylenediamine is polymerised with hexandioic acid to produce nylon, the polymerisation reaction is usually carried out at by mixing together molten hexamethylenediamine and molten hexandioic acid in stoicheiometric amounts. There are many documents discussing this type of process including US-A-4,131,712, WO-A-96/16107 and EP-A-765,855. Also, as described in EP-A-846,754, the process for coating laundry and dishwashing tablets is preferably carried out using molten dicarboxylic acid.

However, the use of molten dicarboxylic acid can be problematic because molten dicarboxylic acids have a tendency to discolour. This discolouration can be undesirable for many applications. For example, it is desirable to consumers that laundry tablets have a high degree of whiteness. When coated with a discoloured dicarboxylic acid coating such tablets appear dirty to the consumer and, therefore, not suitable for laundry purposes.

Various attempts have been made to solve this problem. For example, US-A-4,442,260 describes a process for making a highly concentrated solution of nylon salt which, through controlling water content and reaction temperatures, claims to reduce the rate of the undesirable side reactions that form the coloured by-products. WO-A-99/61510 describes an improved polyamidation system for producing a polyamide from molten dicarboxylic acid monomer and molten diamine monomer. In the system of WO-A-99/61510 oxygen is removed from the solid dicarboxylic acid by use of a vacuum pump and nitrogen displacement. Ultrasonic vibration under a nitrogen atmosphere is then applied to the molten dicarboxylic acid to eliminate further oxygen. This is said to allow the acid to melt without thermal degradation or discolouration.

However, the prior art solutions can be complex, expensive and are not always suitable or successful. Therefore, there exists a need for a simple, effective way of retarding the discolouration of dicarboxylic acids.

The Applicant has surprisingly found that the discolouration of dicarboxylic acids can be retarded by adding water to the molten system.

### Summary of the Invention

According to the present invention there is provided a process for retarding the discolouration of dicarboxylic acids, said process comprising:
(a) heating said dicarboxylic acid to a temperature above its melting point;
(b) adding water to said dicarboxylic acid.

A further aspect of the present invention further provides a composition for retarding the discolouration of dicarboxylic acids, said composition comprising 1g, preferably 2.5g, more preferably 5g, of water per 1,000g of dicarboxylic acid. A further aspect of the present invention provides for the use of water to retard the discolouration of dicarboxylic acids, particularly molten dicarboxylic acids.

Unless otherwise indicated, all ingredients expressed herein are on a weight percentage of the active ingredient.

### Brief Description of the Drawings

Figure 1 shows a process according to the present invention for coating tablets in which the water is added in liquid form to a molten bath of dicarboxylic acid.
Figure 2 shows a process according to the present invention for coating tablets in which the water is added via a steam pipe to a molten bath of dicarboxylic acid.
Figure 3 shows the results, in terms of discolouration as measured by the Hunter-b value, of tablets coated according to the process of Example 1 and Comparative Example 1.
Figure 4 shows the results, in terms of discolouration as measured by the Hunter-b value, of tablets coated according to the process of Example 2 and Comparative Example 2.

### Detailed Description of the Invention

According to the present invention there is provided a process for retarding the discolouration of dicarboxylic acids, said process comprising heating said dicarboxylic acid to a temperature above its melting point and adding water to said dicarboxylic acid. The present process will be described in more detail below.

A further aspect of the present invention provides a composition for retarding the discolouration of dicarboxylic acids, said composition comprising at least 1g, preferably at least 2.5g, more preferably at least 5g, of water per 1,000g of dicarboxylic acid.

A further aspect of the present invention provides for the use of water to retard the discolouration of dicarboxylic acids, particularly molten dicarboxylic acids.

The present use can be with water in any state. However, for practical reasons it is preferred that liquid or gaseous water is used since the addition of ice to the dicarboxylic acid could cause localised solidification of molten dicarboxylic acid.

### Process

The present process comprises the steps:
(a) heating a dicarboxylic acid to a temperature above its melting point;
(b) adding water to said dicarboxylic acid.

Preferably the dicarboxylic acid is heated to a temperature at least 5°C above its melting point, more preferably at least 10°C above its melting point. Therefore, a preferred process comprises the steps:
(a) heating a dicarboxylic acid to a temperature at least 5°C, preferably at least 10°C, above its melting point;
(b) adding water to said dicarboxylic acid.

The water may be added to the dicarboxylic acid at any time but is preferably added after the dicarboxylic acid is molten. Preferably the water is added within 10 minutes, more preferably within 5 minutes, even more preferably within 2 minutes of the dicarboxylic acid becoming molten. Therefore, a preferred process comprises the steps:
(a) heating a dicarboxylic acid to a temperature above its melting point;
(b) adding water to the molten dicarboxylic acid within 10 minutes, preferably within 5 minutes, more preferably within 2 minutes of the dicarboxylic acid becoming molten.

The water may be added in any manner. However, the water is preferably added as liquid or as steam. The water may be added in a single portion, in aliquots or as a continuous stream. For practical purposes the water is preferably added as a continuous stream.

A surprising aspect of the present invention is that a proportionally small amount of water can be effective at retarding the discolouration of a much larger amount of dicarboxylic acid. In the present process the water is preferably added in an amount of at least 1g per 1,000g of dicarboxylic acid. More preferably the water is added in an amount of at least 2.5g per 1,000g of dicarboxylic acid. Even more preferably the water is added in an amount of at least 5g per 1,000g of dicarboxylic acid.

The processes according to the present invention are often carried out at a temperature above the boiling point of water. Therefore, the water added in the present process evaporates. If evaporation of the water occurs, it is necessary to add more water to continue to retard the discolouration of the dicarboxylic acid. Therefore, it is preferred that at least 1g of water per 1,000g of dicarboxylic acid is added per minute. More preferably at least 2.5g of water per 1,000g of dicarboxylic acid is added per minute. Even more preferably at least 5g of water per 1,000g of dicarboxylic acid is added per minute.

Therefore, the preferred process of the present invention comprises the step:
(a) heating a dicarboxylic acid to a temperature at least 5°C, preferably at least 10°C, above its melting point;
(b) adding water to the molten dicarboxylic acid within 10 minutes, preferably within 5 minutes, more preferably within 2 minutes of the dicarboxylic acid becoming molten.
wherein at least 1g, preferably at least 2.5g, more preferably at least 5g, of water per 1,000g of dicarboxylic acid is added per minute.

Preferably the dicarboxylic acid for the present process is selected from C₂-C₁₃ dicarboxylic acids and mixtures thereof, more preferably C₆-C₁₂ dicarboxylic acids and mixtures thereof. Preferred for use herein are hexanedioic acid, octanedioic acid, decanedioic acid, dodecanedioic acid and mixture thereof. More preferred for use herein is hexanedioic acid.

In the present process, water may be added to the dicarboxylic acid in any state but, for practical reasons, it is preferably added as a liquid or as a gas, more preferably as a liquid. The water preferably has a temperature of at least 10°C, more preferably at least 20°C, even more preferably at least 50°C before it is added to the dicarboxylic acid.

### Composition

The compositions of the present invention comprise molten dicarboxylic acid and water. They may comprise other, optional ingredients. However, if present, the compositions herein preferably comprise less than 10%, more preferably less than 5%, by weight, of optional ingredients. The present compositions comprise at least 1g of water per 1,000g of dicarboxylic acid. More preferably the present compositions comprise at least 2.5g of water per 1,000g of dicarboxylic acid. Even more preferably the present compositions comprise at least 5g of water per 1,000g of dicarboxylic acid.

### Dicarboxylic Acids

Any dicarboxylic acid or mixture of dicarboxylic acids may be used herein. However, preferably the dicarboxylic acid is selected from C₂-C₁₃ dicarboxylic acids and mixtures thereof, more preferably C₆-C₁₂ dicarboxylic acids and mixtures thereof. Preferred for use herein are hexanedioic acid, decanedioic acid, octanedioic acid, dodecanedioic acid and mixture thereof. More preferred for use herein is hexanedioic acid. Preferably the dicarboxylic acids for use herein have a melting point of from 80°C to 200°C, more preferably from 90°C to 180°C, even more preferably from 95°C to 160°C.

### Water

Water is the preferred solvent for use herein. Water may be added to the dicarboxylic acid in any state but, for practical reasons, it is preferably added as a liquid or as a gas, more preferably as a liquid. The water preferably has a temperature of at least 10°C, more preferably at least 20°C, even more preferably at least 50°C before it is added to the dicarboxylic acid.

### Preferred Embodiments

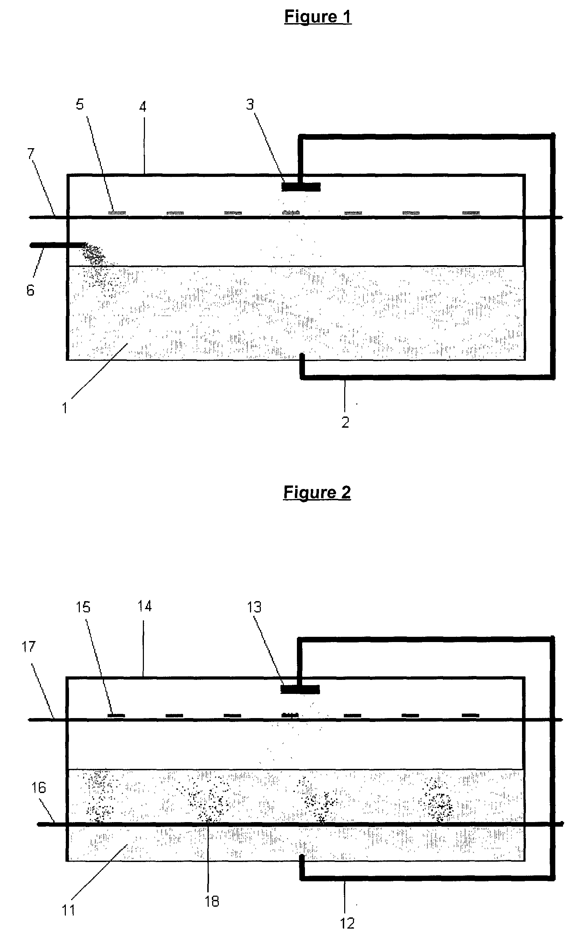

A preferred embodiment of the present invention is shown in Figure 1. In Figure 1, dicarboxylic acid is added to container (4) and then heated with mixing until molten. Water is added to the molten dicarboxylic acid (1) through an inlet pipe (6) at a rate of 30g/min. The molten mixture is then pumped through a pipe (2) to a shower head (3). The shower head is positioned over a conveyer belt (7) carrying detergent tablets (5). The molten dicarboxylic acid is then sprayed over the tablets. The tablets are thus coated with a dicarboxylic acid coating. The excess coating is blown off and the tablets are transported to the outside of the box.

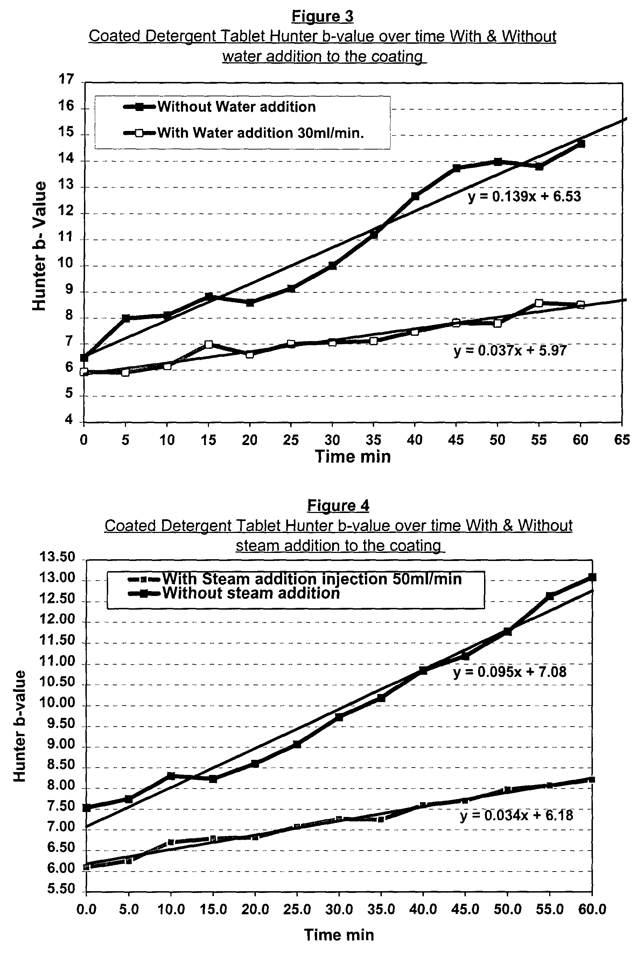

Another preferred embodiment of the present invention is shown in Figure 2. In Figure 2, dicarboxylic acid is added to container (14) and then heated with mixing until molten. Steam is added to the molten dicarboxylic acid (11) through an inlet pipe (16) at a rate of 50g/min. Apertures (18) are spaced along the length of inlet pipe (16) to allow the steam to escape into the molten mixture. The molten mixture is then pumped through a pipe (12) to a shower head (13). The shower head is positioned over a conveyer belt (17) carrying detergent tablets (15). The molten dicarboxylic acid is then sprayed over the tablets. The tablets are thus coated with a dicarboxylic acid coating. The excess coating is blown off and the tablets are transported to the outside of the box.

### Optional Ingredients

The compositions herein can comprise other optional ingredients. A variety of optional ingredients may be useful. These include chelants such aminophosphonates and other phosphonates, hydroxycarboxylic acids such as citric acid, oximes such as dimethylglyoxime, aminocarboxylates, polymers such as polyethyleneimines, ion exchange resins such as PUROLITE™ (available from Purolite International Ltd, Rhondda-Cynon-Taff, UK), integrity improving polymers such as OPADRY™ (available from Colourcon Ltd, Dartford, Kent, UK), -OH donators, heat absorbers such as silicone oil or plasticisers such as terephtalate.

Preferably, chelants are added to the molten dicarboxylic acid. Preferably the chelants are selected from phosphonate chelants, especially hydroyethane diphosphonic acid. While not wishing to be bound by theory, it is believed that the chelants trap heavy metals which would otherwise be free to catalyse degradation and discolouration reactions.

### Examples

The following examples further illustrate the preferred embodiments within the scope of the present invention. The examples are given solely for the purposes of illustration and are not to be construed as limitations of the present invention as many variations of the invention are possible without departing from its spirit or scope.

### Example 1

9.5Kg of hexanedioic acid was mixed with 0.5Kg of PUROLITE™ in a container and then heated to a temperature of 165°C with mixing. Once the mixture was molten, water was added through an inlet pipe at a rate of 30g/min. This molten mixture was pumped through a pipe to a shower head. The shower head was positioned over a conveyer belt carrying detergent tablets. The tablets are thus coated with a hexanedioic acid coating. The excess coating is blown off and the tablets are transported to the outside of the box.

Sample tablets were collected every 5 minutes for a 1 hour period and the level of discoloration, as measured by the Hunter-b value, was assessed. The discolouration was assessed using a Minolta Chroma Meter DP301 with a CR310 Lens. The Chroma Meter was calibrated against a white calibration plate and then the tip of measuring head was placed flat against the centre of the tablet surface. The results are shown in Figure 3.

### Comparative Example 1

Detergent tablets were coated in the exactly the same way as in Example 1 except that no water was added to the molten mixture. Again, sample tablets were collected every 5 minutes for a 1 hour period and, using the above described method, the level of discoloration, as measured by the Hunter-b value, was assessed.

The results are shown in Figure 3. Since the temperature of the system is kept at around 165°C the water quickly evaporates. However, it can be seen that the addition of water to the system retards the rate of discolouration of the molten hexanedioic acid.

### Example 2

9.6Kg of hexanedioic acid was mixed with 0.3Kg of PUROLITE™ in a container and then heated to a temperature of 165°C with mixing. Once the mixture was molten steam was added through an inlet pipe at a rate of 50g/min. Holes were spaced along the length of inlet pipe to allow the steam to escape into the molten mixture. This molten mixture was pumped through a pipe to a shower head. Said shower head was positioned over a conveyer belt carrying detergent tablets. The tablets are thus coated with a hexanedioic acid coating. The excess coating is blown off and the tablets are transported to the outside of the box.

Sample tablets were collected every 5 minutes for a 1 hour period and, using the above described method, the level of discoloration, as measured by the Hunter-b value, was assessed. The results are shown in Figure 4.

### Comparative Example 2

Detergent tablets were coated in the exactly the same way as in Example 2 except that no steam was added to the molten mixture. Again, sample tablets were collected every 5 minutes for a 1 hour period and, using the above described method, the level of discoloration, as measured by the Hunter-b value, was assessed.

The results are shown in Figure 4. It can be seen that the addition of steam to the system retards the rate of discolouration of the molten hexanedioic acid.

| | **Example 3 (% by weight)** | **Example 4 (% by weight)** | **Example 5 (% by weight)** |
|---|---|---|---|
| Hexanedioic Acid | 95.000 | 97.000 | - |
| Dodecanedioic acid | - | - | 98 |
| Water | 0.002 | 0.005 | 0.003 |
| HEDP¹ | 0.01 | 0.01 | 0.01 |
| Purolite² | 4.997 | 4.994 | 4.996 |

| | | | |
|---|---|---|---|
| ¹hydroyethane diphosphonic acid | | | |
| ²available from Purolite International ltd, Rhondda-Cynon-Taff, UK | | | |

## Claims

1. A process for treating dicarboxylic acids, said process comprising:
(a) heating said dicarboxylic acid to a temperature above its melting point;
(b) adding water to said dicarboxylic acid.

2. A process according to Claim 1 wherein the dicarboxylic acid is selected from C₂-C₁₃ dicarboxylic acids and mixtures thereof, preferably C₆-C₁₂ dicarboxylic acids and mixtures thereof, more preferably hexanedioic acid.

3. A process according to Claim 1 or 2 wherein the water is added within 10 minutes, more preferably within 5 minutes, even more preferably within 2 minutes, of the dicarboxylic acid becoming molten.

4. A process according to any of the preceding claims wherein water is added in an amount of at least 1g, preferably at least 2.5g, more preferably at least 5g, per 1,000g of dicarboxylic acid.

5. A process according to any of the preceding claims wherein at least 1g, preferably at least 2.5g, more preferably at least 5g, of water per 1,000g of dicarboxylic acid is added per minute.

6. A process according to any of the preceding claims wherein the dicarboxylic acid is heated to a temperature at least 5°C, preferably at least 10°C, above its melting point.

7. Use of water to retard the discolouration of molten dicarboxylic acids.

8. A use according to Claim 7 wherein the dicarboxylic acid is selected from C₂-C₁₃ dicarboxylic acids and mixtures thereof, preferably C₆-C₁₂ dicarboxylic acids and mixtures thereof, more preferably hexanedioic acid.

9. A composition comprising molten dicarboxylic acid and water **characterised in that** said composition comprises at least 1g, preferably at least 2.5g, more preferably at least 5g, of water per 1,000g of dicarboxylic acid.

10. A composition according to Claim 9 wherein the dicarboxylic acid is selected from C₂-C₁₃ dicarboxylic acids and mixtures thereof, preferably C₆-C₁₂ dicarboxylic acids and mixtures thereof, more preferably hexanedioic acid.

11. A composition according to Claim 8 or 9, wherein the composition additionally comprises a chelant.

12. A process for coating detergent tablets with a dicarboxylic acid, wherein said dicarboxylic acid is retarding from discolouring, said process comprising:
(a) heating a dicarboxylic acid to a temperature above its melting point;
(b) adding water to said dicarboxylic acid; and
(c) applying the dicarboxylic acid to the tablet.
